(19) Europäisches Patentamt
European Patent Office
Office européen des brevets

(11) Numéro de publication : **0 018 692**
**B1**

(12) **FASCICULE DE BREVET EUROPÉEN**

(45) Date de publication du fascicule du brevet : **11.05.83**

(51) Int. Cl.³ : **C 07 C179/10**, C 07 C179/12, C 07 C178/00

(21) Numéro de dépôt : **80200397.0**

(22) Date de dépôt : **29.04.80**

(54) **Procédé pour l'épuration de solutions organiques de peracides carboxyliques.**

(30) Priorité : **04.05.79 FR 7911234**

(43) Date de publication de la demande : **12.11.80 Bulletin 80/23**

(45) Mention de la délivrance du brevet : **11.05.83 Bulletin 83/19**

(84) Etats contractants désignés : **AT BE CH DE FR GB IT LI LU NL SE**

(56) Documents cités :
**FR A 2 211 447**
**FR A 2 379 520**
**FR A 2 381 752**

**Le dossier contient des informations techniques présentées postérieurement au dépôt de la demande et ne figurant pas dans le présent fascicule.**

(73) Titulaire : **PROPYLOX (Société Anonyme)**
**12, avenue de la Renaissance**
**B-1040 Bruxelles (BE)**

(72) Inventeur : **Hardy, Nicolas**
**Rue de la Taille, 52**
**B-5790 Jemeppe-sur-Sambre (BE)**
Inventeur : **Dancot, Georges**
**Rue de l'Aise, 69**
**B-5790 Jemeppe-sur-Sambre (BE)**

(74) Mandataire : **Meyers, Liliane et al**
**Solvay & Cie Département de la propriété industrielle**
**310, rue de Ransbeek**
**B-1120 Bruxelles (BE)**

**EP 0 018 692 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé pour l'épuration de solutions organiques de peracides carboxyliques

La présente invention concerne un procédé pour l'épuration de solutions organiques de peracides carboxyliques et plus particulièrement de solutions organiques de peracides carboxyliques obtenus par réaction des acides ou anhydrides carboxyliques correspondants avec le peroxyde d'hydrogène en présence d'un catalyseur tel que l'acide sulfurique.

Il est connu de fabriquer des solutions organiques de peracides carboxyliques en faisant réagir dans un dispositif d'extraction liquide-liquide une solution aqueuse d'acide sulfurique et de peroxyde d'hydrogène avec une solution organique de l'acide carboxylique correspondant et de laver la solution organique de peracide ainsi obtenue au moyen d'une solution aqueuse très concentrée en acide sulfurique (demande de brevet français n° 78.12254 déposée le 24 avril 1978 au nom d'INTEROX CHEMICALS LTD). Cette épuration a pour but de réduire la teneur en peroxyde d'hydrogène de la solution organique obtenue. Cependant, elle présente l'inconvénient de donner une solution organique de peracide dont la teneur en acide sulfurique est assez élevée ce qui peut favoriser la corrosion et les réactions secondaires lors de l'utilisation ultérieure de ces solutions organiques par exemple pour l'époxydation des oléfines.

Il est également connu de fabriquer des solutions dans le benzène de peracides carboxyliques en faisant réagir en phase aqueuse un acide carboxylique avec du peroxyde d'hydrogène en présence d'un catalyseur acide, en extrayant le peracide du mélange ainsi obtenu au moyen de benzène et en lavant la solution organique de peracide ainsi obtenue au moyen d'eau ou d'acide sulfurique dilué à 25 % (brevet belge 808 108 déposé le 30 novembre 1973 au nom de DEGUSSA). Ce procédé présente l'inconvénient de donner une solution organique de peracide dont la teneur en eau est trop importante ce qui favorise également la corrosion et les réactions secondaires lors de l'utilisation ultérieure de ces solutions organiques, par exemple pour l'époxydation des oléfines.

L'invention a pour but de fournir un procédé d'épuration qui permet d'obtenir des solutions organiques de peracides carboxyliques qui ne contiennent plus que des quantités négligeables d'eau, de peroxyde d'hydrogène et d'acide sulfurique, et qui ne présentent donc pas les inconvénients cités ci-dessus. Le procédé selon l'invention présente l'avantage supplémentaire d'être particulièrement aisé à réaliser. De plus, il ne nécessite pas de consommation d'énergie élevée et n'entraîne que des pertes faibles en peroxyde d'hydrogène. Enfin, la séparation de la solution organique de peracide des solutions de lavage mises en œuvre est aisée même lorsque des solvants organiques lourds, comme les hydrocarbures chlorés, sont utilisés.

L'invention concerne à cet effet un procédé pour l'épuration de solutions organiques de peracides carboxyliques dans un solvant organique inerte provenant de la fabrication des peracides carboxyliques par réaction des acides ou anhydrides carboxyliques correspondants avec le peroxyde d'hydrogène en présence de catalyseurs selon lequel on soumet les solutions à épurer à un lavage au moyen d'une solution aqueuse diluée contenant de 35 à 60 % en poids d'acide sulfurique.

De bons résultats ont été obtenus en utilisant selon l'invention des solutions aqueuses diluées contenant de 40 à 55 % en poids d'acide sulfurique. Les meilleurs résultats ont été obtenus en utilisant des solutions aqueuses diluées contenant environ 50 % en poids d'acide sulfurique.

Selon un mode de réalisation préférentiel de l'invention, le lavage de la solution organique de peracide au moyen de la solution aqueuse diluée est précédé d'un lavage préalable au moyen d'une solution aqueuse concentrée contenant plus de 65 %, et de préférence de 70 à 97 %, en poids d'acide sulfurique.

Les solutions aqueuses d'acide sulfurique utilisées selon l'invention et éventuellement pour le lavage préalable peuvent contenir, outre l'acide sulfurique et l'eau, de petites quantités de produits tels que le peracide carboxylique souhaité et l'acide carboxylique correspondant. Ces produits se trouvent en quantités variables ne dépassant en général pas 20 %, et le plus souvent inférieures à 15 %, du poids de la solution. Elles peuvent également contenir de petites quantités de peroxyde d'hydrogène ; dans ce cas on veille en général à ce que la quantité de peroxyde d'hydrogène présente dans la solution ne dépasse pas 5 % du poids de la solution de manière à éviter la réextraction du peroxyde d'hydrogène par la solution organique. La présence de petites quantités de peroxyde d'hydrogène dans les solutions aqueuses d'acide sulfurique présente l'avantage de réduire la corrosion dans l'installation. Cet effet favorable est déjà obtenu pour des concentrations de 0,01 % en poids de peroxyde d'hydrogène dans la solution aqueuse. On préfère cependant que la concentration soit supérieure à 0,1 % en poids. De bons résultats ont été obtenus avec des solutions aqueuses d'acide sulfurique contenant éventuellement jusqu'à 2 % de leur poids de peroxyde d'hydrogène.

Le procédé selon l'invention convient particulièrement bien pour l'épuration de solutions organiques de peracides carboxyliques obtenus par réaction des acides ou anhydrides carboxyliques correspondants avec le peroxyde d'hydrogène en présence d'acide sulfurique comme catalyseur et extraction, simultanée ou subséquente, des peracides formés par le solvant organique inerte. Dans un tel procédé, en effet, on recueille, à la fin de la fabrication du peracide, d'une part, une solution organique de peracide à épurer et, d'autre part, une solution aqueuse diluée contenant de l'acide sulfurique et éventuellement de petites quantités de peroxyde d'hydrogène non transformé et de peracide et acide carboxyliques

extraits. Cette solution aqueuse diluée d'acide sulfurique peut avantageusement être mise en œuvre, en tout cas en partie, pour le lavage selon l'invention lorsque ce dernier fait suite à un lavage préalable réalisé à l'aide d'une solution aqueuse concentrée d'acide sulfurique. La teneur en acide sulfurique de la solution aqueuse diluée est éventuellement ajustée à la valeur désirée par addition d'eau ou d'acide sulfurique concentré à la solution aqueuse acide recueillie à la fin de la fabrication du peracide.

Dans ce cas, la solution organique de peracide est soumise d'abord à un lavage préalable au moyen d'une solution aqueuse concentrée d'acide sulfurique et la solution aqueuse d'acide sulfurique recueillie à la sortie du lavage préalable peut être renvoyée avantageusement à la fabrication du peracide. La solution organique de peracide issue du lavage préalable est ensuite soumise au lavage selon l'invention au moyen de la solution aqueuse diluée d'acide sulfurique recueillie à la sortie de la fabrication de peracide. Quant à la partie de la solution aqueuse diluée d'acide sulfurique recueillie à la sortie de la fabrication de peracide qui n'a pas été utilisée, elle peut être concentrée et la solution aqueuse concentrée d'acide sulfurique ainsi obtenue peut être mise en œuvre pour réaliser la réaction du peroxyde d'hydrogène avec les acides ou anhydrides carboxyliques correspondants.

Le mode de réalisation du procédé selon l'invention décrit ci-dessus convient tout particulièrement bien pour l'épuration de solutions organiques de peracides carboxyliques obtenues selon le procédé décrit dans la demande de brevet français 78.12254 précitée où la fabrication du peracide et son extraction par le solvant organique inerte ont lieu simultanément.

Une variante préférée de ce mode de réalisation consiste donc à appliquer le procédé selon l'invention à l'épuration de solutions organiques de peracides carboxyliques obtenues par formation et extraction simultanée des peracides formés par le solvant organique inerte, à ajouter à la solution aqueuse concentrée obtenue lors du lavage préalable un appoint de peroxyde d'hydrogène et éventuellement de l'acide sulfurique, et à mettre en œuvre la solution ainsi obtenue pour réaliser la réaction du peroxyde d'hydrogène avec les acides ou anhydrides carboxyliques correspondants.

Dans le mode de réalisation décrit ci-dessus, on met en œuvre de préférence, pour le lavage préalable, une solution aqueuse concentrée d'acide sulfurique contenant moins de 1 % en poids de peroxyde d'hydrogène.

Les quantités de solutions aqueuses d'acide sulfurique à mettre en œuvre pour le lavage et le lavage préalable éventuel sont variables.

La solution aqueuse diluée, est en général mise en œuvre à raison de 0,01 à 100 % et de préférence de 0,1 à 10 % du poids de la solution organique de peracide à traiter.

En général, la quantité de solution aqueuse diluée d'acide sulfurique mise en œuvre est telle que la concentration en acide sulfurique dans la solution aqueuse recueillie après le lavage avec cette solution diluée est comprise entre 35 et 70 % en poids. De bons résultats sont obtenus quand cette concentration est comprise entre 40 et 65 %.

La solution concentrée est mise en œuvre en général à raison de 0,1 à 200 % et de préférence de 1 à 100 % du poids de la solution organique de peracide à traiter.

La température à laquelle est effectuée le lavage de la solution organique de peracide est en général assez basse, le plus souvent comprise entre 263 et 303 K et de préférence entre 268 et 293 K. L'emploi de températures basses permet une réduction de la teneur en eau de la solution organique de peracide.

La température à laquelle est effectuée le lavage préalable ne paraît pas critique en elle-même. En général, on utilise des températures comprises entre 263 et 323 K, et de préférence entre 268 et 303 K.

Les lavages peuvent se faire selon les diverses techniques connues en elles-mêmes. On peut ainsi mettre en contact la solution organique de peracide avec la solution d'acide sulfurique dans des dispositifs d'extraction liquide-liquide tels que des mélangeurs-décanteurs, éventuellement combinés avec des coalesceurs, et des colonnes d'extraction à contre-courant ou à co-courant. On peut aussi utiliser une combinaison d'un ou plusieurs mélangeurs décanteurs, d'une ou de plusieurs colonnes de même type ou de types différents ou encore de plusieurs de ces dispositifs. De bons résultats sont obtenus en utilisant des colonnes d'extraction à contre-courant ou des batteries de mélangeurs décanteurs disposées de manière à exercer un effet de contre-courant.

Les appareils utilisés pour effectuer les lavages sont réalisés dans des matériaux résistant à la corrosion. On peut ainsi utiliser des appareils dont les parois en contact avec la solution organique de peracide et les solutions d'acide sulfurique sont réalisés en acier inoxydable, en acier émaillé, en alliages INCONEL, HASTELLOY, INCOLOY, MONEL, NIMONIC, NI-RESIST et CHLORIMET.

Le procédé selon l'invention peut être appliqué à l'épuration de solutions organiques de divers types de peracides carboxyliques, seuls ou en mélange. Ainsi, il peut être utilisé pour épurer des solutions organiques d'acides mono- ou poly-percarboxyliques. Le procédé convient particulièrement bien pour épurer des solutions organiques de peracides carboxyliques contenant de 1 à 10 atomes de carbone, que ce soient des peracides carboxyliques aliphatiques, alicycliques ou aromatiques, tels que l'acide performique, l'acide peracétique, les peracides chloroacétiques, l'acide perpropionique, les peracides chloropropioniques, l'acide perbutanoïque, l'acide percaproïque, l'acide permaléique, l'acide perheptanoïque, l'acide perbenzoïque, l'acide percyclohexanoïque, l'acide perpélargonique et les acides perphtaliques. Des résultats particulièrement avantageux sont obtenus lors de l'épuration de solutions organiques d'acide peracétique et perpropionique.

Le procédé selon l'invention peut être appliqué à l'épuration de solutions organiques de peracides

carboxyliques dans divers types de solvants inertes. En général, on met en œuvre des solvants peu solubles dans l'eau et dans l'acide sulfurique ; leur solubilité dans ces produits est en général inférieure à 1 % en poids. Ils sont choisis parmi les solvants inertes vis-à-vis des constituants du système dans les conditions de lavage. Ces solvants sont en général choisis parmi les esters carboxyliques, les éthers, les hydrocarbures halogénés, les hydrocarbures non subtitués, les hydrocarbures substitués par des groupes nitro, les esters d'acide nitrique et les esters non acides d'acides carbonique et phosphorique, les nitriles et leurs mélanges.

Comme hydrocarbures halogénés convenant bien en général, on peut signaler les hydrocarbures halogénés aromatiques, aliphatiques et alicycliques contenant de 1 à 8 atomes de carbone dans leur molécule substitués par au moins un halogène choisi de préférence parmi le chlore, le fluor et le brome. Des hydrocarbures halogénés convenant particulièrement bien sont le tétrachlorure de carbone, le chloroforme, le chlorure de méthylène, le chloréthane, les di-, les tri-, les tétra- et le penta-chloréthanes, les trichlorotrifluoréthanes, le tri- et le tétra-chloréthylène, les mono-, les di-, les tri- et les tétra-chloropropanes, les butanes, méthylpropanes, pentanes et hexanes mono- ou poly-chlorés, le mono- et les dichlorobenzènes et les chlorotoluènes.

Parmi tous ces hydrocarbures chlorés on choisit en général les hydrocarbures chlorés inférieurs contenant de 1 à 3 atomes de carbone.

Comme esters carboxyliques convenant bien en général, on peut signaler les esters aliphatiques, alicycliques ou aromatiques d'acides mono- ou poly-carboxyliques avec des alcools mono- ou polyhydriques contenant de 2 à 20 et de préférence de 3 à 10 atomes de carbone dans la molécule. Parmi ceux-ci, conviennent particulièrement bien les formiates et acétates d'isopropyle, de propyle, et butyle, d'isobutyle, de sec-butyle, de tert-butyle, d'amyle, d'isoamyle et de sec-amyle, les mono- et di-chloroacétates, propionates, butyrates et isobutyrates de méthyle, d'éthyle, de propyle, d'isopropyle, de butyle, d'isobutyle et d'isoamyle, les valérates, isovalérates et caproates de méthyle, d'éthyle et de propyle, les acétates de méthoxyéthyle, d'éthoxyéthyle et de cyclohexyle, le pivalate de méthyle, les esters diéthyliques des acides phtalique et adipique, et le phtalate de di-n-butyle.

Comme éthers convenant bien en général, on peut signaler les éthers aliphatiques ou alicycliques symétriques ou asymétriques contenant de 3 à 20 et de préférence de 4 à 12 atomes de carbone tels que le diéthyl éther, le 2,2'-dichlorodiéthyl éther, le méthylpropyl éther, l'éthylpropyl éther, le butyléthyl éther, le tert-butyléthyl éther, le tert-amylméthyl éther, le diisopropyl éther, le dipropyl éther, le dibutyl éther, l'éthylhexyl éther, le diisobutyl éther, le dioxane et le méthylal.

Comme hydrocarbures non substitués convenant bien en général, on peut signaler les hydrocarbures contenant de 5 à 14 atomes de carbone aliphatiques, aromatiques, ou alicycliques, tels que le benzène, le toluène, le xylène, le pentane, l'hexane, l'heptane, l'octane, le diisobutyle, le décane, le cyclohexane, le méthylcyclohexane, la tétraline ou des mélanges d'hydrocarbures aliphatiques tels que l'éther de pétrole.

Comme hydrocarbures substitués par des groupes nitro convenant bien en général, on peut mentionner les hydrocarbures contenant de 1 à 10 et de préférence de 1 à 8 atomes de carbone aromatiques, aliphatiques ou alicycliques, tels que les nitropropanes, le nitrobenzène et le nitrocyclohexane.

Comme esters d'acide carbonique convenant bien en général, on peut mentionner les esters aliphatiques contenant de 3 à 9 atomes de carbone dans la molécule tels que les carbonates de diméthyle, diéthyle, diisobutyle, dibutyle, di-tert-butyle, dipropyle et diisopropyle. Des esters d'acide nitrique convenant bien en général sont ceux choisis parmi les esters aliphatiques contenant de 1 à 5 atomes de carbone dans la molécule tels que les nitrates de méthyle, propyle, butyle et isoamyle.

Quant aux esters d'acide phosphorique convenant bien, ce sont ceux qui répondent à la formule

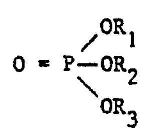

où $R_1$, $R_2$ et $R_3$ sont identiques ou différents et représentent des groupes alkyles, aryles, arylalkyles ou alkylaryles tels que la molécule contient de 3 à 30 atomes de carbone. A titre d'exemples de phosphates particuliers, on peut signaler les phosphates de triméthyle, de tributyle, de trioctyle et de dioctylphényle.

Comme nitriles convenant bien en général, on peut mentionner les nitriles contenant de 2 à 10 atomes de carbone dans la molécule tels que le benzonitrile.

Les solvants les plus fréquemment employés dans les solutions organiques d'acides peracétique et perpropionique sont le benzène, le toluène, le cyclohexane, le décane, l'heptane, l'éther de pétrole, le 1,2-dichloropropane, le 1,1,2,2-tétrachloréthane, le 1,2-dichloréthane, le pentachloréthane, le trichloréthylène, le tétrachloréthylène, le nitrobenzène, le chlorobenzène, le chlorure de cyclohexyle, les phtalates de diéthyle, et de di-n-butyle, le propionate d'éthyle, l'éther di-n-propylique et le phosphate de tri-butyle. Des résultats particulièrement bons sont obtenus avec le 1,2-dichloréthane, le 1,2-dichloropropane, le benzène et leurs mélanges.

Les solutions organiques de peracides carboxyliques à épurer peuvent contenir des quantités variables de peracides carboxyliques. En général, la teneur en peracides des solutions est supérieure à

1 % et de préférence supérieure à 2 % en poids. La concentration maximale en peracide ne dépasse pas, s'il y a lieu, la limite de solubilité du peracide dans le solvant, ni la concentration correspondant à la limite d'explosibilité de la solution. Les solutions organiques de peracides à épurer contiennent en général de 2 à 50 % et le plus souvent de 4 à 40 % en poids de peracides.

Les solutions organiques de peracides carboxyliques à épurer peuvent contenir également des quantités variables de l'acide carboxylique correspondant. Ces acides sont en général présents en quantités comprises entre 0,1 et 60 % et le plus souvent entre 0,5 et 50 % du poids des solutions.

Les impuretés éliminées essentiellement par le procédé selon l'invention sont le peroxyde d'hydrogène, l'eau et l'acide sulfurique. Ces impuretés sont présentes en quantités variables dans les solutions à épurer. En général, les solutions organiques de peracide contiennent

— jusqu'à 1 % en poids de peroxyde d'hydrogène,
— jusqu'à 1 % en poids d'eau et
— jusqu'à 1 % en poids d'acide sulfurique.

Le procédé selon l'invention permet d'obtenir des solutions organiques épurées de peracides carboxyliques convenant particulièrement bien pour l'époxydation d'oléfines telles que le propylène, le chlorure d'allyle, l'alcool allylique et le styrène.

Le procédé selon l'invention peut être réalisé en continu dans des appareils tels que ceux représentés schématiquement aux figures 1 et 2 des dessins en annexe qui se rapportent à des modes de réalisation pratiques particuliers.

Selon le procédé représenté à la figure 1, on introduit dans le réacteur 1 par la voie 2 une solution d'acide carboxylique (AC) dans un solvant organique inerte (SOLV). Par la voie 3 on introduit une solution aqueuse de peroxyde d'hydrogène frais, dans un des courants d'acide sulfurique envoyé au réacteur soit dans la voie 6 (représenté à la figure 1) soit dans la voie 8 (non représenté) soit encore dans la voie 7 (non représenté). La solution aqueuse diluée d'acide sulfurique recueillie au pied du réacteur par la voie 4 peut être partiellement recyclée par les voies 5 et 6 au réacteur 1. Par les voies 7 et 8 on envoie au réacteur des solutions concentrées en acide sulfurique de recyclage, un appoint d'acide sulfurique concentré frais se fait par la voie 9.

La solution organique de peracide à épurer, contenant du peroxyde d'hydrogène, quitte le réacteur 1 par la voie 10 et pénètre dans la zone de lavage préalable 11. La solution d'acide sulfurique concentrée de lavage est introduite dans la zone de lavage préalable 11 par la voie 12. La solution d'acide sulfurique recueillie à la sortie de la zone de lavage 11 passe par la voie 13 et est recyclée par la voie 6 dans le réacteur 1. On recueille à la sortie de la zone de lavage préalable 11, une solution organique de peracide substantiellement exempte de peroxyde d'hydrogène dissous mais contenant de l'acide sulfurique. Cette solution organique, après addition éventuelle d'une fraction de solvant (non représentée), est envoyée par la voie 14 dans la zone de lavage 15 où on introduit par la voie 16 une partie de la solution aqueuse diluée d'acide sulfurique recueillie au pied du réacteur 1 par les voies 4 et 17. On recueille par la voie 18 la solution organique de peracide (PAC) épurée. La solution aqueuse d'acide sulfurique issue de la zone de lavage 15 est recyclée par la voie 7 au réacteur 1.

Une partie de la solution aqueuse diluée d'acide sulfurique recueillie au pied du réacteur 1 est envoyée par les voies 4, 17 et 19 dans une zone de concentration 20 de manière à éliminer l'eau par la voie 21 ; une purge peut être prévue par exemple par la voie 22. La solution aqueuse concentrée d'acide sulfurique est envoyée partiellement par les voies 23 et 8 au réacteur 1 et partiellement par les voies 23 et 12 à la zone de lavage préalable.

La figure 2 représente une variante du procédé représenté à la figure 1 dans laquelle la zone de lavage préalable 11 et le réacteur sont superposés. Les solutions d'acide sulfurique provenant respectivement de la zone de concentration 20 par les voies 23 et 8, de la zone de lavage 15 par la voie 7 et de la zone de réaction 1 par les voies 4 et 6 sont envoyées simultanément par la voie 24 en même temps que le peroxyde d'hydrogène introduit par la voie 3 dans la zone de réaction 1. Dans un tel dispositif on peut également prévoir une introduction étagée le long de la zone de réaction du peroxyde d'hydrogène (non représenté).

Afin d'illustrer l'invention, sans pour autant en limiter la portée, on donne ci-après un exemple pratique de réalisation.

## Exemple

Fabrication d'une solution d'acide perpropionique dans le 1,2-dichloropropane

L'appareil est semblable à celui schématisé à la figure 1.

La zone de lavage préalable 11 est constituée par une colonne d'extraction liquide-liquide à contre-courant.

La zone de lavage 15 comporte un mélangeur, suivi d'un réfrigérant à 273 K et, ensuite, d'un coalesceur-décanteur.

La composition des flux de matière dans les deux zones de lavage est donnée au tableau 1, ci-après en kg/h.

Tableau 1

| | solution aqueuse d'acide sulfurique | | | solution organique de peracide | | |
|---|---|---|---|---|---|---|
| | lavage préalable | lavage | | lavage préalable | | lavage |
| | entrée | entrée | sortie | entrée | sortie / entrée | sortie |
| | voie 12 | voie 16 | voie 7 | voie 10 | voie 14 | voie 18 |
| acide propionique | - | 0,5 | 0,71 | 55,3 | 49,4 | 49,7 |
| acide perpropionique | - | 0,07 | 0,66 | 53,3 | 50 | 48,6 |
| 1,2-dichloropropane | - | - | - | 275,2 | 275,2 | 275,2 |
| eau | 6,81 | 2,85 | 2,81 | 1,4 | 0,6 | 0,63 |
| $H_2O_2$ | 0,02 | 0,05 | 0,28 | 1,1 | 0,34 | 0,34 |
| $H_2SO_4$ | 20,6 | 3,46 | 4,75 | 0,2 | 1,5 | 0,11 |
| $H_2SO_5$ | 0,07 | 0,07 | 0,19 | - | - | - |

La composition en % en poids de la solution organique d'acide perpropionique dans le 1,2-dichloropropane avant et après épuration est donnée au Tableau 2 ci-après.

Tableau 2

| | Composition de la solution organique de peracide, % poids | | |
|---|---|---|---|
| | avant épuration | Après le lavage préalable avec $H_2SO_4$ à 75 % | Après le lavage avec $H_2SO_4$ à 50 % |
| acide propionique | 14,3 | 13,1 | 13,3 |
| acide perpropionique | 13,8 | 13,3 | 13,0 |
| $H_2O$ | 0,36 | 0,16 | 0,17 |
| $H_2O_2$ | 0,285 | 0,09 | 0,09 |
| $H_2SO_4$ | 0,052 | 0,4 | 0,03 |

**Revendications**

1. Procédé pour l'épuration de solutions organiques de peracides carboxyliques dans un solvant organique inerte provenant de la fabrication des peracides carboxyliques par réaction des acides ou anhydrides carboxyliques correspondants avec le peroxyde d'hydrogène en présence de catalyseurs, comportant un lavage de ces solutions au moyen d'une solution aqueuse d'acide sulfurique, caractérisé en ce qu'on soumet les solutions à épurer à un lavage préalable au moyen d'une solution aqueuse concentrée contenant plus de 65 % en poids d'acide sulfurique, et ensuite à un lavage au moyen de 0,1 à 10 % du poids de la solution organique, d'une solution aqueuse diluée contenant de 35 à 60 % en poids d'acide sulfurique et de 0,01 à 2 % en poids de peroxyde d'hydrogène.

2. Procédé selon la revendication 1, caractérisé en ce que la solution aqueuse concentrée d'acide sulfurique utilisée pour le lavage préalable contient moins de 1 % en poids de peroxyde d'hydrogène.

3. Procédé selon la revendication 2, caractérisé en ce qu'il est appliqué à l'épuration de solutions organiques de peracides carboxyliques obtenues par réaction des acides ou anhydrides carboxyliques correspondants avec le peroxyde d'hydrogène en présence d'acide sulfurique comme catalyseur et

extraction des peracides carboxyliques formés par le solvant organique inerte et en ce que l'on utilise pour le lavage la solution aqueuse diluée d'acide sulfurique provenant de l'extraction.

4. Procédé selon la revendication 3, caractérisé en ce qu'il est appliqué à l'épuration de solutions organiques de peracides carboxyliques obtenues par formation et extraction simultanée des peracides formés par le solvant organique inerte, en ce que l'on ajoute à la solution aqueuse concentrée obtenue lors du lavage préalable un appoint de peroxyde d'hydrogène, et éventuellement d'acide sulfurique, et en ce que l'on met en œuvre la solution ainsi obtenue pour réaliser la réaction du peroxyde d'hydrogène avec les acides ou anhydrides carboxyliques correspondants.

5. Procédé selon la revendication 3 ou 4, caractérisé en ce que l'on n'utilise pour le lavage qu'une partie de la solution aqueuse diluée d'acide sulfurique provenant de l'extraction et en ce que l'on concentre l'autre partie et on met en œuvre la solution ainsi obtenue pour réaliser la réaction du peroxyde d'hydrogène avec les acides ou anhydrides correspondants.

6. Procédé selon l'une quelconque des revendications 1 à 5 caractérisé en ce que l'on opère le lavage au moyen de la solution aqueuse diluée d'acide sulfurique à une température comprise entre 268 et 293 K.

7. Procédé selon l'une quelconque des revendications 1 à 6 caractérisé en ce qu'il est appliqué à l'épuration de solutions organiques de peracides carboxyliques dans un solvant choisi parmi le 1,2-dichloréthane, le 1,2-dichloropropane, le benzène et leurs mélanges.

8. Procédé selon l'une quelconque des revendications 1 à 7 caractérisé en ce qu'il est appliqué à l'épuration de solutions organiques de peracides carboxyliques choisis parmi l'acide peracétique et l'acide perpropionique.

**Claims**

1. Process for the purification of organic solutions or percarboxylic acids in an inert organic solvent, originating from the manufacture of the percarboxylic acids by reaction of the corresponding carboxylic acids or anhydrides with hydrogen peroxide in the presence of catalysts, the said process comprising washing these solutions by means of an aqueous solution of sulphuric acid, characterised in that the solutions to be purified are subjected to preliminary washing by means of a concentrated aqueous solution containing more than 65 % by weight of sulphuric acid, and then to washing by means of a dilute aqueous solution containing from 35 to 60 % by weight of sulphuric acid and from 0,01 to 2 % by weight of hydrogen peroxide, the amount of the said aqueous solution being 0,1 to 10 % of the weight of the organic solution.

2. Process according to Claim 1, characterised in that the concentrated aqueous solution of sulphuric acid used for the preliminary washing contains less than 1 % by weight of hydrogen peroxide.

3. Process according to Claim 2, characterised in that it is applied to the purification of organic solutions of percarboxylic acids obtained by reaction of the corresponding carboxylic acids or anhydrides with hydrogen peroxide in the presence of sulphuric acid as a catalyst, and extraction of the percarboxylic acids formed with the inert organic solvent, and in that the dilute aqueous solution of sulphuric acid originating from the extraction is used for the washing.

4. Process according to Claim 3, characterised in that it is applied to the purification of organic solutions of percarboxylic acids obtained by formation of the peracids and simultaneous extraction of the peracids formed with the inert organic solvent, in that an additional amount of hydrogen peroxide and, if appropriate, of sulphuric acid is added to the concentrated aqueous solution obtained during the preliminary washing, and in that the solution thus obtained is used to carry out the reaction of the hydrogen peroxide with the corresponding carboxylic acids or anhydrides.

5. Process according to Claim 3 or 4, characterised in that only part of the dilute aqueous solution of sulphuric acid originating from the extraction is used for the washing, and in that the other part is concentrated and the solution thus obtained is used to carry out the reaction of the hydrogen peroxide with the corresponding acids or anhydrides.

6. Process according to any one of Claim 1 to 5, characterised in that the washing is carried out by means of the dilute aqueous solution of sulphuric acid at a temperature of between 268 and 293 K.

7. Process according to any one of Claims 1 to 6, characterised in that it is applied to the purification of organic solutions of percarboxylic acids in a solvent chosen from amongst 1,2-dichloroethane, 1,2-dichloropropane, benzene and mixtures thereof.

8. Process according to any one of Claims 1 to 7, characterised in that it is applied to the purification of organic solutions of percarboxylic acids chosen from amongst peracetic acid and perpropionic acid.

**Ansprüche**

1. Verfahren zur Reinigung organischer Percarbonsäurelösungen in einem organischen Lösungsmittel aus der Herstellung der Percarbonsaüren durch Reaktion der entsprechenden Carbonsäuren oder -anhydride mit Wasserstoffperoxid in Gegenwart von Katalysatoren, umfassend ein Waschen dieser

Lösungen mit einer wässerigen Schwefelsäurelösung, dadurch gekennzeichnet, dass man die zu reinigenden Lösungen einem vorlaüfigen Waschen mit einer konzentrierten, über 65 Gew.-% Schwefelsäure enthaltenden wässerigen Lösung und hierauf einem Waschen mit 0,1 bis 10 %, bezogen auf das Gewicht der organischen Lösung, einer verdünnten, 35 bis 60 Gew.-% Schwefelsäure und 0,01 bis 2 Gew.-% Wasserstoffperoxid enthaltenden wässerigen Lösung unterwirft.

2. Verfahren nach Anspruch 1, dadurch gekennzeichnet, dass die für das vorläufige Waschen verwendete konzentrierte wässerige Schwefelsäurelösung unter 1 Gew.-% Wasserstoffperoxid enthält.

3. Verfahren nach Anspruch 2, dadurch gekennzeichnet, dass es auf die Reinigung von organischen Lösungen von Percarbonsäuren angewendet wird, die durch Reaktion der entsprechenden Carbonsäuren oder -anhydride mit Wasserstoffperoxid in Gegenwart von Schwefelsäure als Katalysator und Extraktion der gebildeten Percarbonsäuren durch das organische inerte Lösungsmittel erhalten worden sind, und dass man zum Waschen die aus der Extraktion stammende verdünnte wässerige Schwefelsäurelösung verwendet.

4. Verfahren nach Anspruch 3, dadurch gekennzeichnet, dass es auf die Reinigung von organischen Percarbonsäurelösungen angewendet wird, die durch gleichzeitige Bildung und Extraktion der gebildeten Persäuren mit dem inerten organischen Lösungsmittel erhalten worden sind, und dass man zu der beim vorläufigen Waschen erhaltenen konzentrierten wässerigen Lösung einen Zuschuss an Wasserstoffperoxid und gegebenenfalls an Schwefelsäure zusetzt und dass man die solcherart erhaltene Lösung zur Durchführung der Reaktion des Wasserstoffperoxids mit den entsprechenden Carbonsäuren oder -anhydriden einsetzt.

5. Verfahren nach Anspruch 3 oder 4, dadurch gekennzeichnet, dass man für das Waschen nur einen Teil der aus der Extraktion stammenden verdünnten wässerigen Schwefelsäurelösung verwendet und den anderen Teil konzentriert und die solcherart erhaltene Lösung zur Durchführung der Reaktion des Wasserstoffperoxids mit den entsprechenden Säuren oder Anhydriden einsetzt.

6. Verfahren nach einem der Ansprüche 1 bis 5, dadurch gekennzeichnet, dass man das Waschen mit der verdünnten wässerigen Schwefelsäurelösung bei einer Temperatur zwischen 268 und 293 K ausführt.

7. Verfahren nach einem der Ansprüche 1 bis 6, dadurch gekennzeichnet, dass es auf die Reinigung von organischen Percarbonsäurelösungen in einem Lösungsmittel, ausgewählt unter 1,2-Dichloräthan, 1,2-Dichlorpropan, Benzol und deren Gemischen, angewendet wird.

8. Verfahren nach einem der Ansprüche 1 bis 7, dadurch gekennzeichnet, dass es auf die Reinigung von organischen Lösungen von Percarbonsäuren, ausgewählt unter Peressigsäure und Perpropionsäure, angewendet wird.

FIG 1

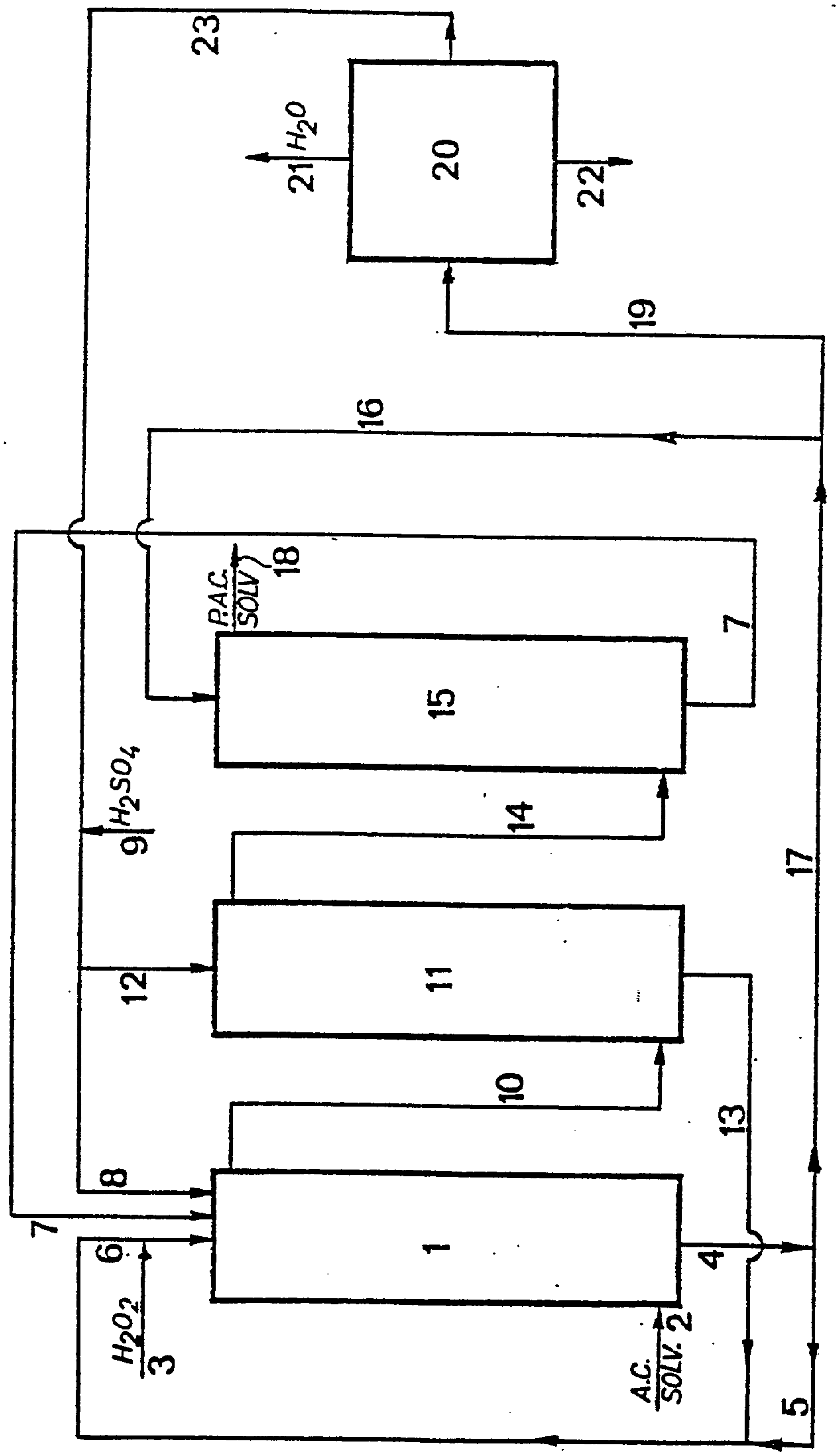

1
2
A.C.
SOLV.
3
H2O2
4
5
6
7
8
9
H2SO4
10
11
12
13
14
15
16
17
18
P.A.C.
SOLV.
19
20
21
H2O
22
23

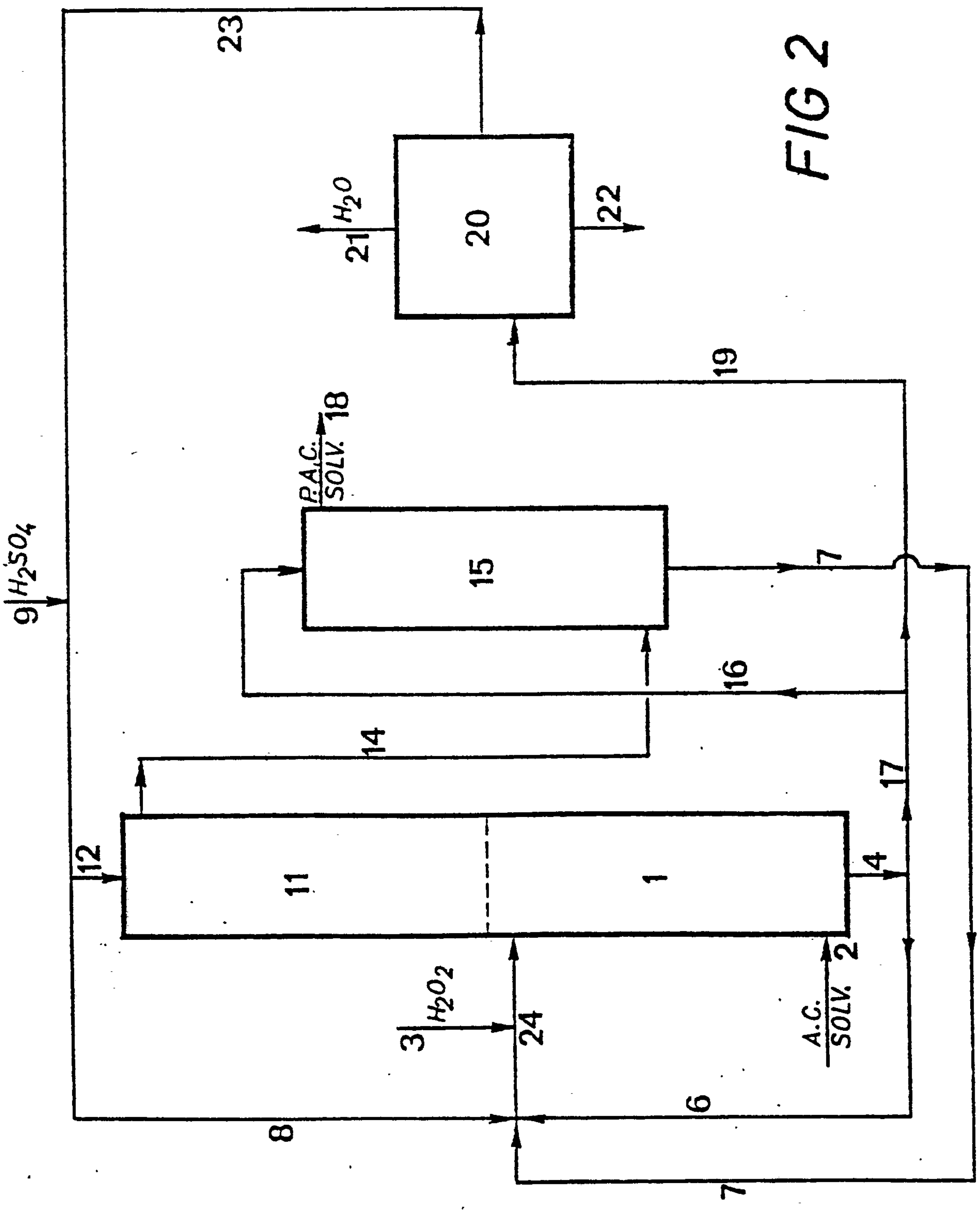
23
20
21
$H_2O$
22
19
P.A.C.
SOLV.
18
15
7
16
14
17
9
$H_2SO_4$
12
11
1
4
2
A.C.
SOLV.
3
$H_2O_2$
24
6
8
7

FIG 2